# EUROPEAN PATENT SPECIFICATION

(11) **EP 1 935 508 B1**
(45) Date of publication and mention of the grant of the patent: **15.12.2010**
(21) Application number: 07018848.7
(22) Date of filing: 25.09.2007
(51) Int. Cl.: B05D 3/10

(54) **Method for producing porous surfaces on metal components**
Verfahren zur Herstellung poröser Oberflächen auf Metallkomponenten
Procédé de production de surfaces poreuses sur des composants métalliques

(30) Priority: 05.12.2006 EP 06025128; 17.04.2007 DE 102007018062
(43) Date of publication of application: 25.06.2008
(73) Proprietor: Linde AG, 80331 München (DE)
(72) Inventor: Laumen, Christoph, 80634 München (DE); Salwén, Anders, Dr., 18262 Djursholm (SE); Wiberg, Sören, 17835 Ekerö (SE)
(74) Representative: Gellner, Bernd

(56) References cited:
- WO-A-01/88972
- US-A1- 2002 198 601
- US-A1- 2005 238 796

## Description

The invention relates to a method for the modification of the surface structure of a metal body, more preferably for increasing the surface porosity.

Stainless austenitic steels after their manufacture frequently have a smooth metal surface without structure and can therefore be frequently wetted only with difficulty. This wetting characteristic has a major influence on the adhesion and durability of paints and coatings, i.e. the application of durable coatings on such surfaces proves difficult.

In medical technology, biocompatible materials such as for example titanium are used for producing implants from these. With such implants, too, a surface that is too smooth also results in problems that are difficult to solve. Among these are for example poor contact between the implant and the human tissue in which the implant is implanted.

In the paper "Porous Metal Tubular Support for Solid Oxide Fuel Cell Design", Electrochemical and Solid-State Letters Volume 9, No. 9, Pages A427 to A429, June 2006, a method for the manufacture of a porous nickel tube is described. To this end, the nickel tube is initially oxidized and subsequently reduced in a hydrogen atmosphere.

The document US 2002/198601 describes that a two step method for enhancing the porosity of a metal body comprising the creation of a surface layer comprising oxygen or nitrogen followed by removal of this surface layer by electrolytic reduction.

The formation of nickel pores at certain temperatures and after certain times is assumed to be due to special relations between the thermal stability of the NiO and the diffusion rates of nickel and oxygen atoms in nickel metal and in NiO.

Contrary to the oxidation of nickel, in Fe or Co containing metals, not only one oxide but more oxides of the type MO, M₂O₃ and M₃O₄, with M = Fe or Co, with different thermal stabilities are formed depending on the oxidation temperature. In addition, in Fe-based or Co-based alloys, oxides formed by alloying ellements such as Cr, Mo, Mn, and Si may be formed which make the picture even more complex. During reduction, the diffusion of Fe or Co and of the alloying elements in the matrix and in the oxides creates an extremely complicated picture.

The same arguments as for Fe- or Co-based alloys also apply for other metal alloys with additions of Cr, Fe, Cu, Co, Mo, Mn and Si, and for Ta- and Ti-based alloys.

The object of the present invention therefore is to show a method in order to increase the surface porosity of a metal body of a metal alloy comprising at least one of the metals Fe, Cu, Co, Cr, Ti, Ta, Mo, Mn and Si.

This object is solved through a method for the modification of the surface structure of a metal body, according to claim 1.

Characteristic of the invention is the two-stage nature of the method. In a first method stage a surface layer is created on the metal body which has at least one nonmetallic element or a compound containing one nonmetallic element. In the first method stage, preferably carbon, oxygen, nitrogen, sulphur or phosphorus are installed in the surface layer as nonmetallic elements. The creation of this surface layer need not take place in one step, but can also be carried out in several steps.

After this, a nonmetallic element or a compound containing these nonmetallic elements can be removed again partly or wholly from the surface layer in a second method stage. In this way, vacancies remain in the surface layer which bring with them a porosity of the surface. This second method stage of the removal of the nonmetallic elements from the surface layer can also take place in one or several steps.

According to a preferred embodiment of the invention there is no need for a final treatment of the surface after the above mentioned second method stage. In particular, the surface layer is not subjected to an etching process or a similar process.

In a preferred embodiment the first method stage and/or the second method stage consist of several method steps, wherein in each method step at least one nonmetallic substance is deposited in the surface layer and/or removed from the surface layer. The metal body can for example be initially modified in a treatment atmosphere so that a nonmetallic element is installed in the surface of said body, for example the metal surface is oxidized through controlled reaction with an atmosphere containing oxygen. After this, the metal body is again subjected to the same treatment in order to bring about an additional installation of the nonmetallic element in the surface structure of the metal body, i.e. in the mentioned example the metal surface would be exposed to an additional oxidation reaction.

The same applies to the second method stage: the removal of the nonmetallic components from the surface layer can likewise take place in several steps. Dividing the two method stages of the installation or the removal of the nonmetallic components into several steps is more preferably practical when a larger amount of these nonmetallic substances is to be installed in or removed from this surface, but prolonged or more intensive treatment of the metal body has disadvantageous effects on the metal properties. Through the mentioned division of a method stage into several steps performed in succession a more careful treatment of the metal body is achieved.

The division of a method stage into several steps is also advantageous if a plurality of different substances is to be installed in or removed from the surface structure of the metal body. To this end, the reaction conditions are optimized in a first step such that preferably a certain nonmetallic substance reacts with the surface of the metal body. In a second step the reaction conditions are modified so that another substance is integrated in the surface layer. Additional steps for the controlled modification of the surface can follow. Of course this does not only apply to the installation of the nonmetallic elements in the surface but also to their removal in a second method stage.

Preferentially the two-stage process according to the invention, i.e. the sequence of the first and the second method stage, is carried out repeatedly. This means a porous surface layer is initially created through the deposition and the at least partial removal again of the mentioned substances. The surface of the metal body pretreated in this way is then preferentially subjected one more time to the method according to the invention under changed method conditions. As a result, a surface with different size pores can be produced. For example a coarsely structured surface, which has inner surfaces with a fine structure, can be created through the appropriate choice of the method parameters. Such surfaces with coarse and fine porosities are for example of advantage for the manufacture of heat exchanger surfaces or catalytic converter surfaces.

The deposition of the nonmetallic elements or compounds containing these and/or their removal from the surface layer is preferentially carried out through treatment of the metal body in a heat treatment atmosphere. By suitably selecting the composition of the heat treatment atmosphere and corresponding selection of the process parameters such as for example pressure and temperature it is possible in this way to bring about reactions between the components of the heat treatment atmosphere and the surface of the metal body in a controlled manner and to produce a defined surface layer.

The metal surface is initially oxidized and subsequently reduced. During the oxidation step an oxide layer forms on the metal surface in the known manner. In a second step the oxidized metal surface is now exposed to a reducing atmosphere. During this treatment at least a part of the existing oxides is removed through reduction, wherein corresponding pores remain in the surface.

By carrying out oxidation followed by reduction of the metal surface a porous surface structure that can be easily wetted is created.

The type and shape of the surface porosity created depends on the method parameters during the first method stage and during the second method stage. In order to obtain a pore structure which is optimally adapted to the set requirements the parameters, more preferably temperature and duration, have to be suitably selected in both the method stages.

These parameters depend on the type of the metal or the alloy whose surface is to be modified, on the substance that is to be installed in or removed from this surface layer, on the type of the oxidizing and/or reduction agent employed and on the desired pore structure, for example their size. If the surface modification through heat treatment of the metal body according to the invention is performed in a gas atmosphere, the temperature of the atmosphere and the respective exposure times are therefore adjusted to the surface to be treated as a function of the type of the metal or the alloy, as a function of the type of the oxidation and reduction agent employed and/or as a function of the desired pore structure.

It has been shown that during the treatment of steels the first method stage is preferably carried out at a temperature between 800°C and 1300 °C, especially preferably between 1000 °C and 1200 °C. For the duration of the heat treatment preferentially a time span between 10 and 200 minutes, preferentially between 30 and 120 minutes, is selected. For titanium, Cr-Co or other materials the mentioned parameters can serve as a first point of reference but further optimization is practical as a rule.

The second method stage, the reduction step, is preferably carried out at temperatures between 900 °C and 1400 °C for the duration of 5 minutes to 120 minutes, more preferably for a duration between 60 and 120 minutes.

To create an oxide layer in the first method stage the surface is advantageously exposed to an atmosphere with an oxygen component of 1 to 100%. Preferentially air is used as oxidizing agent. Depending on the metal to be oxidized and the desired shape of the pore structure however it may also be favourable to carry out the oxidation with the help of air enriched with oxygen or technically pure oxygen as oxidizing agent. Preferably an atmosphere with an oxygen content of at least 50%, especially preferably at least 90% of oxygen is used here. If applicable, the temperature of the oxidation treatment has to be suitably adjusted in this case. It has also been shown also shown that other oxidizing agents such as for example moist air, steam, carbon dioxide or mixtures of nitrogen and oxygen are suitable to bring about the oxidation according to the invention.

In an embodiment of the invention the oxidation of the surface can likewise develop or be created as a side effect during another heat treatment or transformation step.
During hot rolling, for instance, the surface of the metal is already oxidized so that additional separate oxidation need not necessarily be performed.

The reduction of the oxide layer is carried out in a hydrogen atmosphere. Here it has proved itself to employ a reducing atmosphere with hydrogen content of at least 75%, preferably at least 90%, especially preferably at least 98% of hydrogen. Instead or in addition to hydrogen, an atmosphere containing CO can also be used for reduction.

It has been shown that with the treatment of Co-Cr alloys with a relatively high carbon content according to the invention a part of the carbon atoms present in the alloy is utilized in the second method stage for the reduction of the nonmetallic substances. The observed characteristic of carbon as reduction agent can for example be deliberately employed in that carbon is added to the hydrogen or CO atmosphere used in the second method stage.

In a preferred embodiment of the invention the oxidation is carried out in air and the subsequent reduction in an atmosphere of pure hydrogen.

The objective of the method according to the invention is to create a porous metal surface. To this end, non-metals are initially deposited in the surface of the metal body during the first method stage, for example an oxidation step, and, during the second method stage, for example during a reduction step, are removed again so that the desired pores remain. Both these method stages are advantageously carried out in immediate succession. More preferably it is of advantage if the surface is not exposed to any other heat treatment process between the two method stages, more preferably between an oxidation and a reduction step.

The method is used to advantage in order to treat the surface of a body of stainless austenitic steel, a Co-Cr alloy, a nickel alloy, titanium, tantalum or an alloy containing these substances according to the invention. Here, either the entire body or only the surface of the body to be treated is successively exposed to the two method stages, i.e. for example initially to an oxidizing and subsequently to a reducing atmosphere.

The invention is preferably utilized for modifying the surface structure of devices for medical or pharmaceutical purposes. For example medical implants e.g. tooth implants, stents, doctor's instruments, catheters, prostheses or artificial joints or materials of which such implants and prostheses are manufactured, are modified according to the invention.

The surface porosity improves the contact between the implant and the human or animal tissues or bones. On the other hand, the implants or prostheses, but also doctor's instruments, are frequently provided with coatings, for example an hydroxyapatite coating. Through the use of the method according to invention clearly improved adhesion of such layers is achieved.

More preferably in the area of medical technology and surgery, but also in other technical areas, can the pores formed according to the invention be utilized in order to deposit active substances, isotopes, radioactive substances for combating cancer or pharmaceuticals in said pores which are to be given off to the environment or introduced in the surrounding tissue.

Another preferred area of application of the method according to the invention is the treatment of metal surfaces in order to improve their adhesion characteristics for subsequent painting or coating.

Additional areas of application of the invention are the modification of the surface structure of heat exchangers in order to improve the heat transfer and the flow conditions along the heat exchanger surfaces. The modified surfaces according to the invention can also bring advantages in catalytic converters and batteries.

It has also been shown that the optical properties of surfaces, for example the absorption capacity, can be influenced in a controlled manner through the invention. A potential area of application for this are solar collectors.

At present, surfaces requiring a defined structure or porosity are frequently produced through powder coating or siritering-on of powder. The present invention constitutes a cost-effective possibility of superseding these relatively expensive methods.

Finally it is also possible with thin metal bodies to not only modify their surface but to produce a body according to the invention which is porous throughout. Such porous metal bodies for example can be employed as filters.

It has been shown that during the two method stages of the creation or the removal of the nonmetallic components increased diffusion of alloying elements into the metal surface occurs. Advantageously the method according to the invention is therefore utilized to alloy the metal surface in a controlled manner. Especially preferably a few micrometer thick surface layer is created on the metal body in this manner, in which the Cr or Mo-content compared with the remainder of the surface layer is increased. It was for example discovered that the Cr-content in this outermost layer can be increased by 5 to 15%.

This controlled enrichment of elements in the outermost surface layer has more preferably great advantages in such applications where the corrosion resistance of the surface is of great importance, for example in order to protect medical implants from acids produced by the body.

A further preferred area of application of the invention is the increase of the surface hardness, more preferably of micromechanical or electronic components. With the suitable selection of the process parameters a surface layer with particularly small grain size is formed. This is attributed to the fact that the metal atoms which remain after the removal of the nonmetallic atoms in the second method stage compose themselves into new grains. If, in the process, very many new small grains per unit area are formed, this results in a high surface hardness.

The invention has numerous advantages compared with the prior art. With the method according to the invention the surface porosity can be customized. Depth and size of the pores can be set through suitable selection of the method parameters in the oxidation and in the reduction steps. More preferably stainless austenitic steels, Co-Cr-alloys, titanium and tantalum materials, which frequently have a smooth surface structure, can be prepared according to the invention so that subsequent coatings last better and durably.

The invention and additional details of the invention are explained in more detail in the following by means of the exemplary embodiments presented in the drawings. Here the figures show exposures of metal surfaces treated according to the invention.

### Example 1:

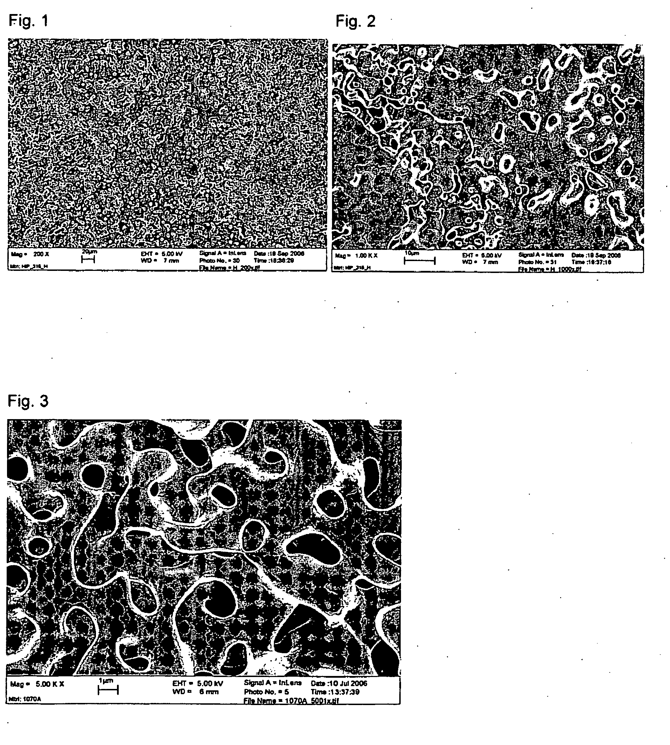

A steel of Type AISI316 was oxidized in an oxygen atmosphere at 1200 °C for 30 minutes and subsequently reduced in a 100% hydrogen atmosphere at 1150°C likewise for 30 minutes. This produced pores with a size between 1 micrometre and 10 micrometres and the pore channels that formed reached a depth of several micrometres. Figures 1 and 2 show exposures of the pores created.

### Example 2:

Figure 3 shows the enlarged representation of a hot-rolled wire whose surface oxidized during hot rolling and which was subsequently reduced in a hydrogen atmosphere at 1170°C. The porosity of the surface is clearly visible.

### Example 3:

The surface porosity of tooth implants of titanium was modified according to the invention. It was discovered that the surface topography of the tooth implants has a substantial influence on the process and the speed of biological processes following the implantation in the human or animal body. This applies to processes in the nanometre range up to processes at the macro-level or with macro-particles.

## Claims

1. A method for increasing the surface porosity of a metal body, wherein the metal body is a metal alloy comprising at least one of the metals Fe, Cu, Co, Cr, Ti, Ta, Mo, Mn and Si as primary component or as addition, and wherein in a first method stage the metal body is treated in a heat treatment atmosphere and on a surface of the metal body a surface layer is created which contains at least one nonmetallic substance, more preferably C, O, N, S or P, and wherein the surface layer is created by reaction between components of the heat treatment atmosphere and the surface of the metal body and wherein subsequently in a second method stage at least one of the nonmetallic substances contained in the surface layer is at least in part removed from the surface layer, **characterized in that** the metal body in the second method stage is modified in an atmosphere containing hydrogen and/or carbon monoxide as reduction agent.

2. The method according to claim 1, **characterized in that** the first method stage and/or the second method stage consists of several method steps, wherein in each method step at least one nonmetallic substance is deposited in the surface layer and/or removed from the surface layer.

3. The method according to any one of the claims 1 or 2, **characterized in that** the sequence of the first and the second method stage are carried out repeatedly.

4. The method according to any one of the claims 1 to 3, **characterized in that** the surface of the metal body in the first method stage is exposed to an oxidizing atmosphere and in the second method stage to a reducing atmosphere.

5. The method according to claim 4, **characterized in that** the metal body in the first method stage is oxidized in an atmosphere containing oxygen, water and/or carbon dioxide as oxidizing agent.

6. The method according to claim 5, **characterized in that** the oxidizing atmosphere has an oxygen content of at least 50%, preferably of at least 75%, particularly preferably of at least 90%.

7. The method according to any one of claims 1 to 6, **characterized in that** the reducing atmosphere has a hydrogen content of at least 75%, preferably of at least 90%, particularly preferably of at least 99%.

8. The method according to any one of the claims 1 to 7, **characterized in that** the surface in the first method stage is treated for a period of time between 10 and 200 minutes preferentially between 30 and 120 minutes.

9. The method according to any one of the claims 1 to 8, **characterized in that** the surface layer in the first method stage is created at a temperature between 800 °C and 1300 °C, preferentially between 1000 °C and 1200 °C.

10. The method according to any one of the claims 1 to 9, **characterized in that** the nonmetallic substances in the second method stage are removed again at least in part at a temperature between 900 °C and 1400 °C, preferentially between 1200 °C and 1300 °C.

11. The method according to any one of the claims 1 to 10, **characterized in that** the surface of a body of stainless austenitic steel, a Co-Cr-alloy, titanium, tantalum or an alloy containing these substances is modified.

12. The method according to any one of the claims 1 to 11, **characterized in that** the surface structure of devices for medical or pharmaceutical purposes, more preferably of implants, is modified.

13. The method according to any one of the claims 1 to 12, **characterized in that** the porosity of the surface is increased and an active substance, more preferably an active substance that is active in a medical or pharmaceutical manner, is deposited in the pores.

14. The method according to any of claims 1 to 13, **characterized in that** the metal alloy is a steel alloyed with chromium or a stainless steel.

## Patentansprüche

1. Verfahren -zur Erhöhung der Oberflächenporosität eines Metallkörpers, wobei es sich bei dem Metallkörper um eine Metallegierung handelt, die mindestens eines der Metalle Fe, Cu, Co, Cr, Ti, Ta, Mo, Mn und Si als Hauptkomponente oder als Zusatz enthält, bei dem der Metallkörper in einer ersten Verfahrensstufe in einer Wärmebehandlungsatmosphäre behandelt und auf einer Oberfläche des Metallkörpers eine Oberflächenschicht erzeugt wird, die mindestens einen nichtmetallischen Stoff, vorzugsweise C, O, N, S oder P, enthält, wobei die Oberflächenschicht durch Reaktion zwischen Komponenten der Wärmebehandlungsatmosphäre und der Oberfläche des Metallkörpers erzeugt wird, und anschließend in einer zweiten Verfahrensstufe mindestens einer der in der Oberflächenschicht enthaltenen nichtmetallischen Stoffe zumindest zum Teil aus der Oberflächenschicht entfernt wird, **dadurch gekennzeichnet, daß** der Metallkörper in der zweiten Verfahrensstufe in einer Wasserstoff und/oder Kohlenmonoxid als Reduktionsmittel enthaltenden Atmosphäre modifiziert wird.

2. Verfahren nach Anspruch 1, **dadurch gekennzeichnet, daß** die erste Verfahrensstufe und/oder die zweite Verfahrensstufe aus mehreren Verfahrensschritten bestehen, wobei in jedem Verfahrensschritt mindestens ein nichtmetallischer Stoff in die Oberflächenschicht eingebracht und/oder aus der Oberflächenschicht entfernt wird.

3. Verfahren nach einem der Ansprüche 1 oder 2, **dadurch gekennzeichnet, daß** die Abfolge der ersten und der zweiten Verfahrensstufe wiederholt durchgeführt wird.

4. Verfahren nach einem der Ansprüche 1 bis 3, **dadurch gekennzeichnet, daß** die Oberfläche des Metallkörpers in der ersten Verfahrensstufe einer oxidierenden Atmosphäre und in der zweiten Verfahrensstufe einer reduzierenden Atmosphäre ausgesetzt wird.

5. Verfahren nach Anspruch 4, **dadurch gekennzeichnet, daß** der Metallkörper in der ersten Verfahrensstufe in einer Sauerstoff, Wasser und/oder Kohlendioxid als Oxidationsmittel enthaltenden Atmosphäre oxidiert wird.

6. Verfahren nach Anspruch 5, **dadurch gekennzeichnet, daß** die oxidierende Atmosphäre einen Sauerstoffgehalt von mindestens 50%, bevorzugt mindestens 75%, besonders bevorzugt mindestens 90%, aufweist.

7. Verfahren nach einem der Ansprüche 1 bis 6, **dadurch gekennzeichnet, daß** die reduzierende Atmosphäre einen Wasserstoffgehalt von mindestens 75%, bevorzugt mindestens 90%, besonders bevorzugt mindestens 99%, aufweist.

8. Verfahren nach einem der Ansprüche 1 bis 7, **dadurch gekennzeichnet, daß** die Oberfläche in der ersten Verfahrensstufe für eine Zeitdauer zwischen 10 und 200 Minuten, vorzugsweise zwischen 30 und 120 Minuten, behandelt wird.

9. Verfahren nach einem der Ansprüche 1 bis 8, **dadurch gekennzeichnet, daß** die Oberflächenschicht in der ersten Verfahrensstufe bei einer-Temperatur zwischen 800°C und 1300°C, vorzugsweise zwischen 1000°C und 1200°C, erzeugt wird.

10. Verfahren nach einem der Ansprüche 1 bis 9, **dadurch gekennzeichnet, daß** die nichtmetallischen Stoffe in der zweiten Verfahrensstufe bei einer Temperatur zwischen 900°C und 1400°C, vorzugsweise zwischen 1200°C und 1300°C, zumindest zum Teil wieder entfernt werden.

11. Verfahren nach einem der Ansprüche 1 bis 10, **dadurch gekennzeichnet, daß** die Oberfläche eines Körpers aus rostfreiem austenitischem Stahl, einer Co-Cr-Legierung, Titan, Tantal oder einer diese Stoffe enthaltenden Legierung modifiziert wird.

12. Verfahren nach einem der Ansprüche 1 bis 11, **dadurch gekennzeichnet, daß** die Oberflächenstruktur von Vorrichtungen für medizinische oder pharmazeutische Zwecke, weiter bevorzugt von Implantaten, modifiziert wird.

13. Verfahren nach einem der Ansprüche 1 bis 12, **dadurch gekennzeichnet, daß** die Porösität der Oberfläche erhöht und ein Wirkstoff, weiter bevorzugt ein medizinisch oder pharmazeutisch wirksamer Wirkstoff, in die Poren eingebracht wird.

14. Verfahren nach einem der Ansprüche 1 bis 13, **dadurch gekennzeichnet, daß** es sich bei der Metallegierung um einen mit Chrom legierten Stahl oder einen rostfreien Stahl handelt.

## Revendications

1. Procédé pour augmenter la porosité de surface d'un corps métallique, le corps métallique étant un alliage métallique comprenant l'un au moins des métaux Fe, Cu, Co, Cr, Ti, Ta, Mo, Mn et Si comme composant principal ou comme additif, dans lequel, dans une première étape de procédé, le corps métallique est traité dans une atmosphère de traitement thermique et une couche superficielle qui contient au moins une substance non métallique, de préférence C, O, N, S ou P, est créée sur une surface du corps métallique, la couche superficielle étant créée par réaction entre les composants de l'atmosphère de traitement thermique et la surface du corps métallique, et dans lequel, par la suite, dans une deuxième étape de procédé, l'une au moins des substances non métalliques contenues dans la couche superficielle est retirée au moins en partie de la couche superficielle, **caractérisé en ce que** le corps métallique à la deuxième étape de procédé est modifié dans une atmosphère contenant de l'hydrogène et/ou du monoxyde de carbone comme réducteur.

2. Procédé selon la revendication 1, **caractérisé en ce que** la première étape de procédé et/ou la deuxième étape de procédé se composent de plusieurs étapes de procédé, dans lequel à chaque étape de procédé au moins une substance non métallique est déposée sur la couche superficielle et/ou retirée de la couche superficielle.

3. Procédé selon l'une quelconque des revendications 1 et 2, **caractérisé en ce que** la séquence de la première et de la deuxième étape de procédé est exécutée de façon répétée.

4. Procédé selon l'une quelconque des revendications 1 à -3, **caractérisé en ce que** la surface du corps métallique est exposée à la première étape de procédé à une atmosphère oxydante et à la deuxième étape de procédé à une atmosphère réductrice.

5. Procédé selon la revendication 4, **caractérisé en ce que** le corps métallique à la première étape de procédé est oxydé dans une atmosphère contenant de l'oxygène, de l'eau et/ou du dioxyde de carbone comme oxydant.

6. Procédé selon la revendication 5, **caractérisé en ce que** l'atmosphère oxydante contient une teneur en oxygène d'au moins 50 %, de préférence d'au moins 75 %, mieux encore d'au moins 90 %.

7. Procédé selon l'une quelconque des revendications 1 à 6, **caractérisé en ce que** l'atmosphère réductrice contient une teneur en hydrogène d'au moins 75 %, de préférence d'au moins 90 %, mieux encore d'au moins 99 %.

8. Procédé selon l'une quelconque des revendications 1 à 7, **caractérisé en ce que** la surface à la première étape de procédé est traitée pendant une période de temps comprise entre 10 et 200 minutes, de préférence entre 30 et 120 minutes.

9. Procédé selon l'une quelconque des revendications 1 à 8, **caractérisé en ce que** la couche superficielle à la première étape de procédé est créée à une température comprise entre 800 °C - et 1300 °C, de préférence entre 1000 °C et 1200 °C.

10. Procédé selon l'une quelconque des revendications 1 à 9, **caractérisé en ce que** les substances non métalliques à la deuxième étape de procédé sont de nouveau retirées au moins en partie à une température comprise entre 900 °C et 1400 °C, de préférence entre 1200 °C et 1300 °C.

11. Procédé selon l'une quelconque des revendications 1 à 10, **caractérisé en ce que** la surface d'un corps en acier inoxydable austénitique, alliage Co-Cr, titane, tantale ou alliage contenant ces substances est modifiée.

12. Procédé selon l'une quelconque des revendications 1 à 11, **caractérisé en ce que** la structure superficielle de dispositifs à des fins médicales ou pharmaceutiques, de préférence d'implants, est modifiée.

13. Procédé selon l'une quelconque des revendications 1 à 12, **caractérisé en ce que** la porosité de la surface est augmentée et une substance active, de préférence une substance qui est active d'une manière médicale ou pharmaceutique, est déposée dans les pores.

14. Procédé selon l'une quelconque des revendications 1 à 13, **caractérisé en ce que** l'alliage métallique est un acier allié au chrome ou un acier inoxydable.
